# EUROPEAN PATENT APPLICATION

(11) **EP 1 413 316 A1**
(43) Date of publication of application: **28.04.2004**
(21) Application number: 02405838.0
(22) Date of filing: 27.09.2002
(51) Int. Cl.: A61K 47/48

(54) **Bifunctional conjugates or fusion proteins**

(71) Applicant: Robert, Bruno, 30170 St Hippolyte du Four (FR); Donda, Alena, 1080 Les Cullayes (CH); Cesson, Valérie, 74500 Saint Gingolph (FR); Mach, Jean-Pierre, 1293 Bellevue (CH)
(72) Inventor: Robert, Bruno, 30170 St Hippolyte du Four (FR); Donda, Alena, 1080 Les Cullayes (CH); Cesson, Valérie, 74500 Saint Gingolph (FR); Mach, Jean-Pierre, 1293 Bellevue (CH)
(74) Representative: Besse, François

(57) **Abstract**

The invention relates to the coupling or to the genetically fusing of two different proteins: one, which has the property of binding selectively to a target cell, such as an antibody or a ligand for a receptor overexpressed on target cells, and one consisting of a monomorphic MHC class I related protein, such as MIC-A or -B, or ULBP, or CD1d. This conjugate or fusion protein is designed to be localized in vivo on the surface of the target cells such as tumor cells or cells involved in neoangiogenesis. The high density of these monomorphic MHC class I related proteins will activate effector cells such as NK, NKT or other T cells with the objective to kill the targeted cells. The advantage of these MHC class I related molecules is that since they are monomorphic, the same conjugate or fusion protein can be used in every patient. Furthermore, the NK or NKT cells being involved in *innate immunity*, the reaction will be more rapid than that of *acquired immunity* and will involve a much higher population than antigen-specific MHC restricted T cells

## Description

### FIELD OF THE INVENTION

The invention relates to chemical conjugates or fusion proteins comprising a molecule binding to target cells and at least one activating protein.

### BACKGROUND AND PRIOR ART

### A. General introduction.

The inventors have recently developed a combined strategy of tumor targeting, using monoclonal antibodies (mAbs) specific for a tumor-associated antigen (TAA), which are coupled to a soluble classical MHC class I molecule loaded with a highly antigenic viral peptide specific for cytotoxic T lymphocytes (CTL). Thus, the CTL are activated and selectively kill the targeted cancer cells.

In a first approach, they have developed tetramers of HLA-A2 loaded with the immunodominant Flu-MA peptide and coupled to either one of 3 different Fab' fragments from mAbs specific for TAA (anti-CEA, anti-ErbB-2 or anti-CD20). It was demonstrated that tumor expressing the relevant TAA were rendered susceptible to efficient lysis by Flu-Ma peptide specific cytotoxic T lymphocytes (1).

In a second step, the inventors have demonstrated that also monomers of Fab-HLA-A2/Flu-peptide conjugates are active, but only when they reach their target and are oligomerized on the surface of the tumor cells (2). This property presents several advantages to the multimerized conjugates also developed by others (3, 4). First, monomers do not induce significant activation in solution and hence can be injected in larger amounts without side effects. Second, technically, it is easier to produce monomeric forms of conjugates or fusion proteins in amounts necessary for patient treatment (see PCT/LTS01/17184 WO 01/90198).

One limitation of MHC class I/ peptide complexes are their polymorphism, which means that a large pool of conjugates should be developed to be able to treat the great majority of patients. That is the reason why in the present invention, we are introducing the so-called non-polymorphic MHC class I related molecules. They differ from MHC class I molecules, not only because they are non-polymorphic, but also because they contain, either no antigenic peptide for the MIC-A/B or ULBP molecules, or a glycolipid antigen for the CD1d molecules. In addition, these class I related molecules activate other types of effector cells belonging to the innate immune response, namely NK and NKT cells.

### B. Introduction to MIC-A/B and ULBP non-polymorphic class I related molecules.

Among the first monomorphic class I related molecules, which have been described, are the MIC-A and -B, which have a high degree of homology between each other (84%) (5, 6). Contrary to the classical MHC class I MHC molecules which form an heterodimer between the heavy chain and the β-2 microglobulin, the MIC-A and -B consist of a single heavy chain, which has only a low degree of homology with the heavy chain of classical MHC class I (about 30%) (7).

The crystal structure of MIC-A has been recently established (6). It revealed a dramatically altered MHC class I fold, both in detail and overall domain organization. There is only a remnant of a peptide binding groove and a loss of β-2 microglobulin binding site.
The expression of MIC-A and -B protein is essentially restricted to gut epithelium and the corresponding genes appear to be under the control of a heat-shock promoter element . Thus, the expression of MIC-A can be induced on the epithelial cells by various stress, including viral infection and malignant transformation (8).

One of the major biological properties of MIC-A and -B is to bind specifically to the NKG2D activating receptor present on NK cells, as well as on CD8 T cells expressing also, either the γ-δ or the α-β antigen receptor. Through this binding to NKG2D receptor, the MIC-A and -B can act as a natural antigen and activate NK and T cells and induce them to kill the epithelial cells overexpressing the MIC-proteins. This activation of effector cells is more rapid than the system of acquired immunity and represents a first line of defence belonging to the innate immunity (8).

ULBPs are a different group of monomorphic MHC class I related proteins, which are GPI-anchored and have only 25% amino acid sequence homology with classical MHC class I molecules and also a low sequence homology with MIC-A or-B (23%) (7). The ULBPs are overexpressed in cells infected with the human cytomegalovirus (HCMV) and they share with MIC-A and -B the property of binding the activating NKG2D receptor. Thus, human cells infected with HCMV through overexpression of ULBPs can stimulate cytokine and chemokine production by NK cells and confer susceptibility to NK cells cytotoxicity (7). In the present strategy, ULBPs conjugated to antibodies are used as ligands for NKG2D receptor and are targeted on tumor cells independently of HCMV infection.

### C. Introduction to CD1 non-polymorphic class I related molecules

The CD1 family represents another non-polymorphic lineage of class I related antigen-presenting molecules. Some T cells recognize bacterial and self-glycolipids associated with CD1 molecules (9-12). Five CD1 monomorphic isoforms have been identified (CD1a, b, c, d and e), which have a high degree of homology among themselves and a lower degree of homology with the classical MHC class I molecules (almost no homology on α1 and up to 35% on α2 and α3 domains). Despite low sequence homology, the resolution of the crystal structure of mouse CD1d1 revealed a folding similar to MHC class I (13). The major difference resides in the antigen binding groove which is composed of only two very hydrophobic and deep pockets, that are likely to accomodate the lipidic tail of the antigen, while presenting the carbohydrate part to the T cell receptor. Numerous reports have described foreign and self-glycolipid presentation by CD1 molecules in bacterial infections and autoimmunity and their role in innate as well as acquired imunity has been established (10-12).

Of importance for the present invention, the CD1d molecule has been broadly associated with anti-tumor immunity (14, 15). Several other characteristics have drawn much attention on CD1d in the past few years which makes this molecule very attractive for our strategy. First, CD1d-restricted T cells are characterized both in mice and humans by a highly restricted TCR repertoire and expression of some NK markers (CD161, NKG2d). They were therefore called NKT cells and are considered as part of innate immunity (16).

Second, NKT cells have an unsual ability of secreting both Th1 and Th2 cytokines and their role in inflammation, autoimmunity and tumor immunity is being extensively investigated (16-18). Last, the natural ligands of CD1d are still largely unknown but a high affinity ligand and potent activator of NKT cells has been isolated from an extract of a marine sponge known in japanese medicine to have antimetastatic effects. This glycosphingolipid absent in mammal tissues was characterized as α-galactosylceramide (α-Galcer) and has been used both *in vitro* and *in vivo* in various tumor models. It was confirmed that a-Galcer antimetastatic activity is mediated by CD1d-restricted NKT cells (14, 15).

### IMMUNOTARGETING MONOMORPHIC MHC CLASS I-RELATED MOLECULES ON TUMOR CELLS OR NEOANGIOGENESIS

It is evident from the above introduction (A-C), that targeting a high density of MHC class I-related molecule such as MIC-A or -B, ULBP or CD1d on the surface of tumor cells will activate different effector cells such as NK, NKT, or T cells and induce them to kill target tumor cells.

The inventors and others have previously shown experimentally and clinically that radiolabeled or fluorescent labeled mAbs directed against TAA can be specifically targeted in vivo on tumor cells (19, 20). Thus our general strategy will be to couple monomorphic MHC class I-related proteins to anti-TAA mAbs or fragments of mAbs in order to target them on tumor cells. The interest of the proposed strategy is that it takes advantage of both the efficient targeting properties of high affinity anti-TAA mAbs and the powerful and rapid activation of effector cells known to play an essential role in *innate immunity.*

### Example 1: Anti-tumor antibody-MIC-A or B or ULBP conjugates or fusion proteins

### a) Chemical conjugate

MIC-A or -B or ULBP molecules are produced in bacteria or in eukariotic cells with a mutation introducing a cysteine at the C-terminus, as described for HLA-A2 by Robert *et al.* (2). Then, the free SH group on the cysteine is saturated with an excess of bismaleimide coupling reagent. After elimination of the excess bis-maleimide, the maleimide derivatized MIC-A/B or ULBP is chemically coupled to the free SH groups of the cysteines from the Fab' fragments of a high affinity mAb anti-TAA, as described (2).

### b) Fusion protein

The genes encoding MIC-A or -B, or ULBP are fused to the genes encoding either a single chain antibody fragment or a Fab fragment directed against a human TAA. The genes are fused so that the synthesis starts either at the N-terminus of the MIC-A/B or ULBP and continues with the antibody fragment or at the N-terminus of the antibody fragment and be followed by the MIC-A/B or ULBP. In both cases, a semi-rigid amino-acid spacer sequence is placed between the two molecules to avoid steric hindrance. The best form of fusion protein is selected by in vitro testing. For these tests the different fusion proteins are incubated on ⁵¹Cr-labeled target tumor cells bearing the relevant TAA and different amounts of NK cells are added. The conjugate, which induces the most efficient lysis, as determined by ⁵¹Cr release is selected.

### c) In vivo evaluation of the conjugate or fusion proteins

The conjugates or fusion proteins which gave the best *in vitro* results in the ⁵¹Cr release assay are then tested *in vivo* first in experimental animal models and subsequently in tumor bearing patients. The experimental animal bearing a tumor known to express the relevant TAA are injected i.v. with increasing amounts of antibody-MIC-A/-B or ULBP conjugates or fusion proteins.

One or two days after injection of the conjugate or fusion proteins, the animal or the patients are treated with a cytokine or chemokine such as interferon-γ or Il-12 or IL-2, known to stimulate NK cells activity. The tumor size is monitored to verify that the tumor cells, which have been targeted with an optimal dose of conjugates or fusion proteins, are selectively killed by NK cells.

### Example 2: Anti-tumor antibody CD1d conjugates or fusion proteins

### a) Chemical coupling

Recombinant soluble human CD1d are produced in eukariot cells with a mutation at the C-terminus introducing a single cysteine residue. After derivation of the free cysteine with an excess of bis-maleimide and elimination of the excess of free bis-maleimide, the maleimide derivatized CD1d is coupled to the free SH group of cysteines from an Fab' fragment of a high affinity anti-TAA mAb, as described previously (2).

### b) Fusion protein

The cDNA of soluble CD1d protein is fused to the sequences encoding scFv or Fab fragment from a high affinity anti-TAA mAb. Two types of fusion will be made so that either the CD-1d will be expressed at the N-terminus, followed by the antibody fragment or the opposite. In both cases, a semi-rigid amino-acid spacer sequence is placed between the antibody fragment and the CD1d molecule to avoid steric hindrance. The most active form of fusion protein is selected in vitro on target cancer cells expressing the relevant TAA, as described in Example 1b, except that the CD1d in the fusion protein is loaded with α-galactosylceramide and that NKT cells are used as effector cells instead of NK cells.

### c) In vivo evaluation of the conjugate or fusion protein

The conjugate or fusion proteins which gave the best *in vitro* results are tested *in vivo,* first in tumor bearing experimental animals and subsequently in patients, as described in example 1c, except that the antibody fragment-CD1d conjugate or fusion protein is loaded with α-galactosylceramide or with different activating glycolipids.

Alternatively, the antibody-CD1d conjugate or fusion protein is injected without α-galactosylceramide or glycolipids, in order that the CD1d molecules, targeted on tumor cells, become progressively loaded with endogenous glycolipids.

### Example 3: Anti-neoangiogenesis antibody-MHC class I related conjugates or fusion proteins

### a) Introduction

It is now broadly accepted that neoangiogenesis represents an essential condition for tumor development and growth. Thus, one application of our strategy consists in antibody targeting of monomorphic MHC class I related protein such as MIC-A/B, ULCBPs or CD1d molecules in the neovessels. The presence of monomorphic MHC class I related molecules in the neovessels will focus NK and NKT cells activity in the tumor area. The activation of NK and NKT cells in the tumor neovessels have three beneficial effects : 1) It increases inflammation in the tumor area and enhances anti-tumor immune response by recruitment of antigen presenting cells and T lymphocytes through local secretion of cytokines and interleukins ; 2) It has a direct cytotoxic effect against endothelial cells from tumor neovessels and thus decreases blood flow in the tumor and 3) The increased secretion of TNF and other cytokines by macrophages ultimately induce the collapse of the tumor neovasculature.
Numerous mAbs directed against antigens associated with tumor neovessels have been described, such as mAbs against αvβ3 integrins, TNF receptors, platelet derived growth factor receptor, or the ED-B oncofoetal domain of fibronection (21) and many others (for review, (22) and are be used in this strategy of tumor treatment.

### b) Chemical coupling and fusion between mAbs against neoangiogenesis antigens and monomorphic MHC class I related proteins.

The synthesis of chemical conjugates and fusion proteins between the mAbs anti-neoangiogenesis antigens and the MICA-A/B, ULBPs or CD1d is performed as described in Examples 1 and 2, paragraph a) and b).

### c) In vitro evaluation of the conjugates or fusion proteins on endothelial cells.

The effect of the different conjugates or fusion proteins is tested on human umbilical vein endothelial cells (HUVEC), or on tumor cell lines expressing neoangiogenesis antigens in the presence of either NK or NKT cells. The target cells are 51Cr-labeled and their lysis by effector cells is measured in presence or absence of conjugate or fusion protein as described for tumor target cells in Examples 1 and 2, c). Alternatively, the degree of activation of NK or NKT cells in presence of target cells coated or not with the conjugates or fusion proteins is evaluated by measurement of the release of cytokines or interleukins such as IFN-γ or IL4 by NK or NKT cells.

### d) In vivo evaluation of conjugates or fusion proteins on tumor bearing experimental animals.

The effect of intravenous injection of conjugates or fusion proteins on tumor growth is evaluated in tumor bearing mice. Furthermore the tumor vascular parameters, such as blood flow, blood volume and vascular permeability is analyzed during conjugate or fusion protein treatment, as previously described for TNF treatment (23).

### REFERENCES

1. Robert, B., P. Guillaume, I. Luescher, P. Romero, and J. P. Mach. 2000. Antibody-conjugated MHC class I tetramers can target tumor cells for specific lysis by T lymphocytes. *Eur J Immunol 30:3165.*
2. Robert, B., P. Guillaume, I. Luescher, M. A. Doucey, J. C. Cerrotini, P. Romero, and J. P. Mach. 2001. Redirecting anti-viral CTL against cancer cells by surface targeting of monomeric MHC class I-viral peptide conjugated to antibody fragment. *Cancer Immunity 1*.
3. Ogg, G. S., P. R. Dunbar, V. Cerundolo, A. J. McMichael, N. R. Lemoine, and P. Savage. 2000. Sensitization of tumour cells to lysis by virus-specific CTL using antibody-targeted MHC class I/peptide complexes. *Br J Cancer 82:1058*.
4. Savage, P., P. Cowbum, A. Clayton, S. Man, T. Lawson, G. Ogg, N. Lemoine, A. McMichael, and A. Epenetos. 2002. Anti-viral cytotoxic T cells inhibit the growth of cancer cells with antibody targeted HLA class l/peptide complexes in SCID mice. *Int J Cancer 98: 561.*
5. Bauer, S., V. Groh, J. Wu, A. Steinle, J. H. Phillips, L. L. Lanier, and T. Spies. 1999. Activation of NK cells and T cells by NKG2D, a receptor for stress-inducible MICA. *Science 285:727.*
6. Li, P., S. T. Willie, S. Bauer, D. L. Morris, T. Spies, and R. K. Strong. 1999. Crystal structure of the MHC class I homolog MIC-A, a gammadelta T cell ligand. *Immunity 10:577.*
7. Cosman, D., J. Mullberg, C. L. Sutherland, W. Chin, R. Armitage, W. Fanslow, M. Kubin, and N. J. Chalupny. 2001. ULBPs, novel MHC class I-related molecules, bind to CMV glycoprotein UL16 and stimulate NK cytotoxicity through the NKG2D receptor. *Immunity 14:123.*
8. Groh, V., R. Rhinehart, H. Secrist, S. Bauer, K. H. Grabstein, and T. Spies. 1999. Broad tumor-associated expression and recognition by tumor-derived gamma delta T cells of MICA and MICB. *Proc Natl Acad Sci U S A 96: 6879.*
9. Porcelli, S. A., and R. L. Modlin. 1999. The CD 1 system: antigen-presenting molecules for T cell recognition of lipids and glycolipids. *Annu Rev Immunol 17:297*.
10. Shamshiev, A., A. Donda, I. Carena, L. Mori, L. Kappos, and G. De Libero. 1999. Self glycolipids as T-cell autoantigens. *Eur J Immunol 29:1667.*
11. Park, S. H., and A. Bendelac. 2000. CD1-restricted T-cell responses and microbial infection. *Nature 406: 788.*
12. Matsuda, J. L., and M. Kronenberg. 2001. Presentation of self and microbial lipids by CD1 molecules. *Curr Opin Immunol 13:19.*
13. Zeng, Z., A. R. Castano, B. W. Segelke, E. A. Stura, P. A. Peterson, and I. A. Wilson. 1997. Crystal structure of mouse CD1: An MHC-like fold with a large hydrophobic binding groove. *Science 277:339.*
14. Wilson, M. T., A. K. Singh, and L. Van Kaer. 2002. Immunotherapy with ligands of natural killer T cells. *Trends Mol Med 8:225.*
15. Brutkiewicz, R. R., and V. Sriram. 2002. Natural killer T (NKT) cells and their role in antitumor immunity. *Crit Rev Oncol Hematol 41:287.*
16. Bendelac, A., O. Lantz, M. E. Quimby, J. W. Yewdell, J. R. Bennink, and R. R. Brutkiewicz. 1995. CD1 recognition by mouse NK1+ T lymphocytes. *Science 268:863.*
17. Chen, H., and W. E. Paul. 1997. Cultured NK1.1+ CD4+ T cells produce large amounts of IL-4 and IFN-gamma upon activation by anti-CD3 or CD1. *J Immunol 159:2240.*
18. Exley, M., J. Garcia, S. P. Balk, and S. Porcelli. 1997. Requirements for CD1d recognition by human invariant Valpha24+ CD4-CD8- T cells. *J Exp Med 186:109.*
19. Delaloye, B., A. Bischof-Delaloye, F. Buchegger, V. von Fliedner, J. P. Grob, J. C. Volant, J. Pettavel, and J. P. Mach. 1986. Detection of colorectal carcinoma by emission-computerized tomography after injection of 1231-labeled Fab or F(ab')2 fragments from monoclonal anti-carcinoembryonic antigen antibodies. *J Clin Invest 77:301.*
20. Folli, S., G. Wagnieres, A. Pelegrin, J. M. Calmes, D. Braichotte, F. Buchegger, Y. Chalandon, N. Hardman, C. Heusser, J. C. Givel, and et al. 1992. Immunophotodiagnosis of colon carcinomas in patients injected with fluoresceinated chimeric antibodies against carcinoembryonic antigen. *Proc Natl Acad Sci U S A 89: 7973.*
21. Halin, C., S. Rondini, F. Nilsson, A. Berndt, H. Kosmehl, L. Zardi, and D. Neri. 2002. Enhancement of the antitumor activity of interleukin-12 by targeted delivery to neovasculature. *Nat Biotechnol 20:264.*
22. Brower, V. 1999. Tumor angiogenesis--new drugs on the block. *Nat Biotechnol 17: 963.*
23. Folli, S., Pèlegrin, A., Chalandon, Y., Xiaofee, Y, Buchegger, F., Lejeune, F.,Lienart, D. and Mach, J.-P. 1993. Tumor Necrosis Factor can enhance radioantibody uptake in human colon carcinoma xenografts by increasing vascular permability. **INt. J Cancer**, 53:829.

## Claims

1. A chemical conjugate or a bifunctional fusion protein comprising an activating protein able of activating the natural killer (NK) cells as well as the NKT lymphocytes and a binding molecule able of targeting specifically the conjugate on a special population of cells, such as tumor cells, in order to sensitize them to the killing action of NK cells or NKT lymphocytes.

2. A chemical conjugate or a bifunctional fusion protein according to claim 1 wherein said activating protein is a monomorphic MHC class I related protein.

3. A bifunctional protein according to claim 2 wherein said monomorphic MHC class I related protein is a MIC-A or MIC-B.

4. A bifunctional protein according to claim 2 wherein said monomorphic MHC class I related protein is selected from the group called ULBPs.

5. A bifunctional protein according to claim 2 wherein said activating protein is a CD1d or a similar protein that, after loading with a glycolipid such as α-galactosyl ceramide or similar molecules, can activate the NK T cells and different T lymphocyte subsets.

6. A bifunctional protein according to anyone of the previous claims wherein said binding molecule is an intact antibody, preferably directed against a tumor associated antigen to which is coupled at least one of said activating protein.

7. A bifunctional protein according to claim 6 in which said binding molecule is an antibody fragment single chain Fv , Fab or other type of fragment .

8. A bifunctional protein according to claims 6 or 7 in which the antibody is designed to be directed against a tissue specific antigen to treat a tumor derived from this tissue

9. A bifunctional protein according to claims 6 or 7 in which the antibody or fragment of antibody is designed to be directed against an antigen associated with neoangiogenesis, in order to activate NK or NKT cells at the site of neoangiogenesis.

10. A bifunctional protein according to anyone of claims 1 to 5 in which said binding molecule is a ligand or a receptor which binds specifically to a receptor or a ligand, respectively, which is overexpressed by the target cells.
